# EUROPÄISCHE PATENTSCHRIFT

(11) **EP 3 986 172 B1**
(45) Veröffentlichungstag und Bekanntmachung des Hinweises auf die Patenterteilung: **23.08.2023**
(21) Anmeldenummer: 20733598.5
(22) Anmeldetag: 17.06.2020
(51) Int. Cl.: A24F 40/10, A24F 40/42, A24F 40/46

(54) **VERDAMPFERKARTUSCHE SOWIE INHALATOR MIT EINER SOLCHEN VERDAMPFERKARTUSCHE**
VAPORIZER CARTRIDGE AND INHALER COMPRISING SUCH A VAPORIZER CARTRIDGE
CARTOUCHE DE VAPORISATION ET INHALATEUR POURVU D'UNE TELLE CARTOUCHE DE VAPORISATION

(30) Priorität: 20.06.2019 DE 102019116726
(43) Veröffentlichungstag der Anmeldung: 27.04.2022
(73) Patentinhaber: Körber Technologies GmbH, 21033 Hamburg (DE)
(72) Erfinder: BERGMANN, Max, 22303 Hamburg (DE); CORNILS, Lasse, 22605 Hamburg (DE); GIESE, Matthias, Tokyo 104-0053 (JP); HANNEKEN, Christian, 22299 Hamburg (DE); JAKLIN, Jan, 70736 Fellbach (DE); KESSLER, Marc, 22415 Hamburg (DE); KLEINE WÄCHTER, Michael, 23881 Lankau (DE); MÜLLER, Thomas, 20259 Hamburg (DE); ROMMING, Niklas, 22085 Hamburg (DE); SCHMIDT, Rene, 21244 Buchholz i.d.N. (DE); SCHUSTER, Christof, 20259 Hamburg (DE); WUTTKE, Tobias, 21465 Reinbek (DE)
(74) Vertreter: Stork Bamberger Patentanwälte PartmbB
(86) Internationale Anmeldenummer: PCT/EP2020/066745
(87) Internationale Veröffentlichungsnummer: WO 2020/254394

(56) Entgegenhaltungen:
- WO-A1-2020/065077
- DE-A1-102016 120 803
- US-A1- 2017 295 844

## Beschreibung

Die Erfindung betrifft eine Verdampferkartusche als Bestandteil eines Inhalators, umfassend einen Gehäusekörper mit einem Strömungskanal, einen Vorratstank zum Bevorraten von Flüssigkeit sowie eine ein Dochtorgan und ein Heizorgan umfassende Verdampfereinheit, die flüssigkeitspermeabel ausgebildet ist, derart, dass Flüssigkeit mindestens initial kapillar aus dem Vorratstank durch die Verdampfereinheit in Richtung des Strömungskanals förderbar ist, wobei der Gehäusekörper mit einem Hohlraum ausgebildet ist, wobei der Hohlraum des Gehäusekörpers zur Bildung des Vorratstanks einerseits und zur Bildung eines Aufnahmeraums für das Dochtorgan andererseits durch eine den Strömungskanal mindestens abschnittsweise aufweisende Zwischenwand in zwei Kammern aufgeteilt ist, wobei in der Zwischenwand mindestens eine Zugangsöffnung vom Aufnahmeraum für das Dochtorgan zum Strömungskanal ausgebildet ist.

Des Weiteren betrifft die Erfindung einen Inhalator, ausgebildet und eingerichtet zum Inhalieren von mit Wirkstoffen angereichertem Dampf, umfassend einen mindestens eine elektronische Steuereinheit und eine Energiequelle umfassenden Kartuschenträger sowie eine Verdampferkartusche.

Solche Verdampferkartuschen und Inhalatoren kommen in der Genussmittelindustrie, hier insbesondere im Zusammenhang mit einer elektronischen Zigarette, der so genannten E-Zigarette, sowie im medizinischen Bereich zum Einsatz, um fluide Genussmittel und/oder fluide medizinische Produkte in Dampfform und/oder als Aerosole inhalieren zu können. Beim Konsumieren saugt üblicherweise eine Person an einem Mundstück des Inhalators, wodurch in dem Strömungskanal ein Saugdruck entsteht, der einen Luftstrom durch den Strömungskanal erzeugt. Der Luftstrom kann aber auch maschinell z.B. durch eine Pumpe erzeugt werden. In dem Strömungskanal wird dem Luftstrom eine von der Verdampfereinheit erzeugte und bereitgestellte verdampfte Flüssigkeit zugegeben, um der konsumierenden Person ein Aerosol oder ein Aerosol-Dampf-Gemisch zu verabreichen. Die Flüssigkeit ist an der oder in der Verdampferkartusche bevorratet. Als Flüssigkeit kommen unterschiedliche Mischungen mit verschiedenen Bestandteilen gleicher oder unterschiedlicher Dampfdichten zum Einsatz. Eine typische Mischung für den Einsatz in einer E-Zigarette weist z.B.

Bestandteile von Glycerin und Propylenglycol auf, ggf. angereichert um Nikotin und/oder nahezu beliebige Geschmacksstoffe. Für den Einsatz im medizinischen oder therapeutischen Bereich, z.B. zur Inhalation von Asthma-Präparaten, kann die Mischung entsprechend medizinische Bestandteile und Wirkstoffe aufweisen.

Die einzelnen Bestandteile der Verdampferkartusche, nämlich der den Strömungskanal aufweisende Gehäusekörper, der Vorratstank und die Verdampfereinheit können in einem gemeinsamen Bauteil zusammengefasst sein, wobei dieses Bauteil dann ein Einwegartikel ist, der für eine endliche Anzahl von Inhalationszügen durch eine konsumierende Person ausgelegt ist und zusammen mit einem Kartuschenträger als wiederverwendbarem Mehrwegartikel, der mindestens eine elektronische Steuereinheit und eine Energiequelle umfasst, einen Inhalator bildet. Die Verdampferkartusche kann jedoch auch erst durch das Zusammenfügen mehrerer Bauteile gebildet sein, wobei einzelne Bauteile, z.B. die Verdampfereinheit oder Teile davon, in dem Kartuschenträger als Mehrwegartikel angeordnet sind, und der Vorratstank als separates Bauteil den Einwegartikel bildet. Letztlich lässt sich der Inhalator durch Austausch des Einwegartikels, der üblicherweise die Flüssigkeit beinhaltet, variabel einsetzen.

Entsprechend sind der Einwegartikel und der Mehrwegartikel lösbar miteinander verbunden. Der Kartuschenträger als Mehrwegartikel umfasst üblicherweise mindestens eine elektronische Steuereinheit und eine Energiequelle. Die Energiequelle kann z.B. eine elektrochemische Einwegbatterie oder ein wiederaufladbarer elektrochemischer Akku, z.B. ein Li-lonen-Akku sein, mittels dem das Heizorgan über elektrische Kontakte der Verdampfereinheit mit Energie versorgt wird. Die elektronische und/oder elektrische Steuereinheit dient zum Steuern der Verdampfereinheit innerhalb der Verdampferkartusche. Der Kartuschenträger kann aber auch Bestandteile der Verdampferkartusche umfassen. Der Einwegartikel kann die Verdampferkartusche oder Teile davon umfassen und als Ansteckteil an den Mehrwegartikel ansteckbar oder als Einsetzteil in den Mehrwegartikel einsetzbar ausgebildet sein. Anstelle einer Steckverbindung sind auch Schraubverbindungen, Schnappverbindungen oder andere lösbare Schnellverbindungen einsetzbar. Mit der Verbindung von Einwegartikel und Mehrwegartikel wird eine mechanische und elektrische Kopplung zur Bildung eines funktionsbereiten Inhalators hergestellt.

Die zentrale und letztlich die Nutzung (z.B. als E-Zigarette oder als medizinischer Inhalator) bestimmende Komponente ist der Vorratstank als Bestandteil der Verdampferkartusche. Diese beinhaltet in der Regel die von der Person gewählte, gewünschte und/oder benötigte Flüssigkeit bzw. ein Flüssigkeitsgemisch (im Folgenden auch allgemein als Fluid bezeichnet) sowie den den Strömungskanal bildenden bzw. aufweisenden Gehäusekörper und die Verdampfereinheit. Das Fluid ist in dem Vorratstank der Verdampferkartusche bevorratet. Mittels der flüssigkeitspermeablen Verdampfereinheit wird das Fluid aus dem Vorratstank aufgrund zumindest initial kapillarer Förderung durch das Dochtorgan und das Heizorgan geleitet. Die von einer Energiequelle erzeugte Spannung, die an dem Heizorgan angelegt wird, führt zu einem Stromfluss im Heizorgan. Aufgrund des Heizwiderstands, vorzugsweise des Ohm'schen Widerstands des Heizorgans führt der Stromfluss zu einer Erhitzung des Heizorgans und letztlich zu einer Verdampfung des in der Verdampfereinheit befindlichen Fluids. Das auf diese Weise erzeugte Gas bzw. Dampf entweicht aus der Verdampfereinheit in Richtung des Strömungskanals und wird durch Vermischung mit der Luftströmung zu Aerosol rekondensiert. Das Fluid hat damit einen vorgegebenen Weg mit einer vorgegebenen Strömungsrichtung, nämlich als Fluid durch das Dochtorgan an das und durch das Heizorgan und als Gas bzw. Dampf und/oder Aerosol aus dem Heizorgan in den Strömungskanal. In dem Strömungskanal wird das verdampfte Fluid durch den Luftstrom rekondensiert und mitgerissen, wenn der Strömungskanal mit einem Druck/Unterdruck beaufschlagt wird, indem z.B. eine konsumierende Person an dem Strömungskanal saugt oder eine Pumpe einen Luftstrom durch den Strömungskanal fördert.

Damit das Fluid aus dem Vorratstank nicht direkt in den Strömungskanal fließt, deckt die Verdampfereinheit den Zugang vom Vorratstank zum Strömungskanal vollständig ab. Vollständig abgedeckt bedeutet in diesem Zusammenhang, dass die Flüssigkeit zwingend durch die Verdampfereinheit geführt ist, so dass das Fluid nicht direkt vom Vorratstank in den Strömungskanal gelangen kann, sondern den "Umweg" über das Dochtorgan und das Heizorgan nehmen muss. Das Dochtorgan dient zum einen zum Zwischenspeichern von Fluid, um insbesondere bei nahezu entleertem Vorratstank noch ausreichend Fluid für wenige Züge am Inhalator zur Verfügung zu stellen. Das Dochtorgan dient zum anderen insbesondere zum Transport des Fluids vom Vorratstank in Richtung des Strömungskanals sowie zur geometrisch gleichmäßigen Versorgung des Heizorgans mit Liquid (synonym für Fluid und Flüssigkeit) und wirkt gleichzeitig als eine Art Rückschlagschutz, um den Rücklauf von Fluid und/oder Dampf bzw. Gas in Richtung des Vorratstanks zu unterbinden.

Bisher bekannte Verdampferkartuschen weisen eine Verdampfereinheit mit einem Dochtorgan auf, der aus mehreren miteinander verwobenen/verdrillten Fäden/Fasern z.B. aus Baumwolle oder aus Glasfasern gebildet ist. Dieser Faserdocht bzw. in Reihe geschaltete Faserdochte weisen kapillare Eigenschaften auf, die dazu führen, dass beim initialen Kontakt mit dem Fluid, der Faserdocht oder die Faserdochte tauchen in den Vorratstank ein, das Fluid in dem Vorratstank aufgenommen und in Richtung des Heizorgans gefördert wird. Das Heizorgan ist üblicherweise in Form einer Glühwendel ausgebildet. Dieser gewickelte Metalldraht besteht beispielsweise aus Edelstahl, Kupfer, Kupferverbindungen oder Nickel. Diese Verdampfereinheit ist in der Regel nur manuell herzustellen und weist eine begrenzte Speicherkapazität zum Zwischenspeichern von Fluid auf. Ein weiterer Nachteil besteht in der geringen Transportrate von Fluid aufgrund der begrenzten Anzahl von Mikrokanälen sowie in der sich prinzipiell über die Längsrichtung des Docht-Wendel-Systems einstellende inhomogene Temperaturverteilung mit dem Risiko lokaler Überhitzung und der daraus resultierenden Schadstoffentstehung. Mit anderen Worten ist eine gleichmäßige und kontinuierliche Versorgung des Heizorgans mit dem Fluid nur begrenzt sichergestellt.

Eine Verdampferkartusche mit den Merkmalen des Oberbegriffes des Anspruches 1 ist in der US 2017/0295844 A1 beschrieben. Die WO 2020/065077 A1 beschreibt ebenfalls eine Verdampferkartusche mit den Merkmalen des Oberbegriffes des Anspruches 1.

Der Erfindung liegt die Aufgabe zugrunde, eine kompakte Verdampferkartusche vorzuschlagen, die für konstante und reproduzierbare Verdampfungsbedingungen eine verbesserte Flüssigkeitsanbindung zwischen Vorratstank und Heizorgan sicherstellt. Die Aufgabe besteht weiterhin darin, einen entsprechenden Inhalator zu schaffen.

Diese Aufgabe wird durch eine Verdampferkartusche der eingangs genannten Art dadurch gelöst, dass die Zugangsöffnung vollständig durch das Heizorgan abgedeckt ist, und in dem Aufnahmeraum eine Vielzahl granulatartiger, lose nebeneinanderliegender Körner zur Bildung des am Heizorgan anliegenden Dochtorgans angeordnet sind, die den Aufnahmeraum vorzugsweise ausfüllen und aufgrund ihrer losen Schüttung und/oder Ausbildung Mikrokanäle bilden und durch ein Deckelelement im Aufnahmeraum gehalten werden, wobei in der Zwischenwand mindestens eine Zugangsöffnung zur Herstellung einer Flüssigkeitsverbindung vom Vorratstank zum Aufnahmeraum ausgebildet ist, die mit einer flüssigkeitspermeablen Gitterstruktur abgedeckt ist.

Vorzugsweise ist der Gehäusekörper mit einem durchgängigen Strömungskanal ausgebildet, der mindestens eine Eintrittsseite Es und mindestens eine Austrittsseite As aufweist, wobei der Gehäusekörper rohrförmig mit einem sich längsaxial erstreckenden Hohlraum ausgebildet ist und durch eine den durchgängigen Strömungskanal mindestens abschnittsweise aufweisende Zwischenwand in zwei längsaxial hintereinander angeordnete Kammern aufgeteilt ist.

Mit Schüttung der Körner ist das lose Nebeneinanderliegen der Körner beschrieben, wobei auch gerüttelte und/oder verdichtete Anordnungen der Körner umfasst sind. Mit Ausbildung der Körner ist z.B. beschrieben, dass die Körner selbst Mikrohohlräume und/oder Mikrokanäle aufweisen können. Somit werden in der Verdampfereinheit zwischen den einzelnen, aneinanderliegenden Körnern und/oder durch einzelne Körner eine Vielzahl zufälliger Mikrokanäle zwischen dem Vorratstank und dem Strömungskanal gebildet, die eine konstante und gleichmäßige Verdampfung auf der Austrittsseite der Verdampfereinheit sicherstellen. Anders ausgedrückt wird durch das granulare Dochtorgan eine optimale Fluidkopplung zwischen der Eintrittsseite in die Verdampfereinheit und der Austrittsseite aus der Verdampfereinheit hergestellt. Ein weiterer wesentlicher Vorteil wird darin gesehen, dass das granulare Material den gesamten Aufnahmeraum oberhalb des Heizorgans ausfüllt, wodurch gegenüber anderen Dochtlösungen ein vergrößertes Docht-Speichervolumen bereitgestellt ist.

Besonders vorteilhaft gestaltet sich die Montage eines granularen Dochtorgans, da sich dieses an dem jeweiligen Montageort an jede beliebige Kontur/Geometrie der Aufnahme des Dochtorgans anpassen kann. Durch die Kornstruktur passt sich das Dochtorgan beim Montieren/Abfüllen des granularen Materials flexibel an die jeweilige Kontur/Geometrie an und füllt nicht Mikrokanäle bildende Hohlräume auf und vermeidet Spaltbildung zu angrenzenden Flächen. Im Ergebnis werden durch das granulare Dochtorgan eine konstante und reproduzierbare Liquidversorgung des Heizorgans und damit konstante und reproduzierbare Verdampfungsbedingungen sicherstellt. Dabei spielt es keine Rolle, ob die Verdampfereinheit - mit dem Dochtorgan und/oder dem Heizorgan als Bestandteil der Verdampferkartusche - an dem oder in dem Kartuschenträger angeordnet ist, also am/im Mehrwegartikel, oder ob die Verdampfereinheit am/im Einwegartikel angeordnet ist.

Vorteilhafterweise ist die Verdampfereinheit Bestandteil eines Mehrwegartikels des Inhalators, während der Vorratstank Bestanteil eines Einmalartikels des Inhalators ist.

Eine bevorzugte Ausführungsform der Verdampferkartusche ist dadurch gekennzeichnet, dass die Zwischenwand ausgehend von einem den Hohlraum teilenden, tellerartigen Abschnitt mit einem vorzugsweise quer zur Längsachse des Hohlraums verlaufenden Abschnitt des Strömungskanals einen durch den Vorratstank verlaufenden kanalartigen Abschnitt mit einem vorzugsweise parallel zur Längsachse des Hohlraums verlaufenden Abschnitt des Strömungskanals zur Bildung eines Schlots aufweist, wobei die beiden Abschnitte des Strömungskanals miteinander verbunden sind und in ihrer Schnittstelle eine Dampfkammer bilden. Neben einer besonders kompakten Bauweise führt diese Ausbildung aufgrund der Dampfkammer zu verbesserten Verdampfungsbedingungen.

Vorteilhafterweise verläuft der vorzugsweise parallel zur Längsachse des Hohlraums verlaufende kanalartige Abschnitt der Zwischenwand zentral durch den Vorratstank, wobei zu beiden Seiten des kanalartigen Abschnitts der Zwischenwand im tellerartigen Abschnitt derselben jeweils eine Zugangsöffnung zur Herstellung einer Fluidverbindung vom Vorratstank zum Aufnahmeraum ausgebildet ist, die mit einer flüssigkeitspermeablen Gitterstruktur abgedeckt ist. Damit ist die Flüssigkeitsverbindung zwischen Vorratstank und Heizorgan optimiert.

Vorzugsweise ist das Heizorgan ein im Wesentlichen aus Silizium bestehendes oder Silizium oder p- oder n-dotiertes Silizium aufweisendes MEMS-Bauteil (Micro-Electro-Mechanical-System), das ausgehend von einer dem Dochtorgan zugewandten Oberseite bis zu einer dem Strömungskanal zugewandten Unterseite flüssigkeits- und dampfpermeable Durchgänge aufweist. Mit diesem platzsparenden Heizorgan ist eine besonders effektive Dampfbildung erzielbar.

Eine bevorzugte Weiterbildung der Verdampferkartusche ist dadurch gekennzeichnet, dass sie zum mechanischen und elektrischen Verbinden mit einem mindestens eine elektronische Steuereinheit und eine Energiequelle umfassenden Kartuschenträger zur Bildung eines Inhalators ausgebildet und eingerichtet ist, wobei die Verdampfereinheit elektrische Kontakte zur elektrischen Kontaktierung mit der Energiequelle umfasst. Dadurch sind kompakte Verdampferkartuschen/Inhalatoren mit konstanten und reproduzierbaren Verdampfungsbedingungen geschaffen.

Die Aufgabe wird auch durch einen Inhalator der eingangs gennannten Art dadurch gelöst, dass die Verdampferkartusche nach einem oder mehreren der Ansprüche 1 bis 7 ausgebildet und eingerichtet ist.

Die sich daraus ergebenden Vorteile wurden bereits im Zusammenhang mit der Verdampferkartusche beschrieben, weshalb zur Vermeidung von Wiederholungen auf die vorstehenden Ausführungen verwiesen wird.

Weitere zweckmäßige und/oder vorteilhafte Merkmale und Weiterbildungen zur Verdampferkartusche und dem Inhalator ergeben sich aus den Unteransprüchen und der Beschreibung. Besonders bevorzugte Ausführungsformen der Verdampferkartusche und des Inhalators werden anhand der beigefügten Zeichnung näher erläutert. In der Zeichnung zeigt:
- Fig. 1: eine schematische Darstellung eines Inhalators mit einer Verdampferkartusche im Schnitt,
- Fig. 2: wesentliche Teile der Verdampferkartusche gemäß Figur 1,
- Fig. 3: die Verdampferkartusche gemäß Figur 2 in einer um 90° um die Längsachse gedrehten Ansicht,
- Fig. 4: die Verdampferkartusche gemäß Figur 3 entlang des Schnitts A-A,
- Fig. 5: eine weitere Ausführungsform einer Verdampferkartusche, und
- Fig.6: die Verdampferkartusche gemäß Figur 5 in einer um 90° um die Längsachse gedrehten Ansicht.

Die in der Zeichnung dargestellte Verdampferkartusche sowie der Inhalator dienen zum Inhalieren von mit Wirkstoffen, z.B. Nikotin, angereichertem Dampf und/oder Aerosolen aus Flüssigkeiten, sind entsprechend im Zusammenhang mit einer E-Zigarette beschrieben. Die Verdampferkartusche und der Inhalator sind in gleicher Weise zum Inhalieren von mit medizinischen Wirkstoffen angereichertem Dampf aus pharmazeutischen und/oder nahrungsergänzenden Produkten einsetzbar.

Die dargestellte Verdampferkartusche 10 als Bestandteil eines Inhalators 11 umfasst einen Gehäusekörper 12 mit einem Strömungskanal 13, einen Vorratstank 14 zum Bevorraten von Flüssigkeit sowie eine ein Dochtorgan 15 und ein Heizorgan 16 umfassende Verdampfereinheit 17, die flüssigkeitspermeabel ausgebildet ist, derart, dass Flüssigkeit mindestens initial kapillar aus dem Vorratstank 14 durch die Verdampfereinheit 17 in Richtung des Strömungskanals 13 förderbar ist, wobei der Gehäusekörper 12 mit einem Hohlraum 18 ausgebildet ist, wobei der Hohlraum 18 des Gehäusekörpers 12 zur Bildung des Vorratstanks 14 einerseits und zur Bildung eines Aufnahmeraums 19 für das Dochtorgan 15 andererseits durch eine den Strömungskanal 13 mindestens abschnittsweise aufweisende Zwischenwand 20 in zwei Kammern 21, 22 aufgeteilt ist, wobei in der Zwischenwand 20 mindestens eine Zugangsöffnung 23 vom Aufnahmeraum 19 für das Dochtorgan 15 zum Strömungskanal 13 ausgebildet ist.

Diese Verdampferkartusche 10 zeichnet sich erfindungsgemäß dadurch aus, dass die Zugangsöffnung 23 vollständig durch das Heizorgan 16 abgedeckt ist, und in dem Aufnahmeraum 19 eine Vielzahl granulatartiger, lose nebeneinanderliegender Körner 24 zur Bildung des am Heizorgan 16 anliegenden Dochtorgans 15 angeordnet sind, die den Aufnahmeraum 19 vorzugsweise ausfüllen und aufgrund ihrer losen Schüttung und/oder Ausbildung Mikrokanäle 25 bilden und durch ein Deckelelement 26 im Aufnahmeraum 19 gehalten werden, wobei in der Zwischenwand 20 mindestens eine Zugangsöffnung 27 zur Herstellung einer Flüssigkeitsverbindung vom Vorratstank 14 zum Aufnahmeraum 19 ausgebildet ist, die mit einer flüssigkeitspermeablen Gitterstruktur 28 abgedeckt ist.

Vorzugsweise ist der Gehäusekörper 12 mit einem durchgängigen Strömungskanal 13 ausgebildet, der mindestens eine Eintrittsseite Es und mindestens eine Austrittsseite As aufweist, wobei der Gehäusekörper 12 rohrförmig mit einem sich längsaxial erstreckenden Hohlraum 18 ausgebildet ist und durch eine den durchgängigen Strömungskanal 13 mindestens abschnittsweise aufweisende Zwischenwand 20 in zwei längsaxial hintereinander angeordnete Kammern 21, 22 aufgeteilt ist.

Der zylindrische Gehäusekörper 12 ist zu einer Stirnseite hin geschlossen ausgebildet, nämlich zu der Stirnseite, an der das Deckelelement 26 aufgesteckt, aufgeschraubt oder anderweitig befestigt ist. Auf der dem Deckelelement 26 abgewandten Stirnseite weist der Gehäusekörper 12 mindestens eine Öffnung 29 auf, die den Austritt As des oder jeden Strömungskanals 13 bildet, der mindestens einen radial gerichteten Eintritt Es in den Gehäusekörper 12 zur Bildung des durchgängigen Strömungskanals 13 aufweist.

Die im Folgenden beschriebenen Merkmale und Weiterbildungen stellen für sich betrachtet oder in Kombination miteinander bevorzugte Ausführungsformen dar. Es wird ausdrücklich darauf hingewiesen, dass Merkmale, die in den Ansprüchen und/oder der Beschreibung und/oder der Zeichnung zusammengefasst oder in einer gemeinsamen Ausführungsform beschrieben sind, auch funktional eigenständig die weiter oben beschriebene Verdampferkartusche 10 weiterbilden können.

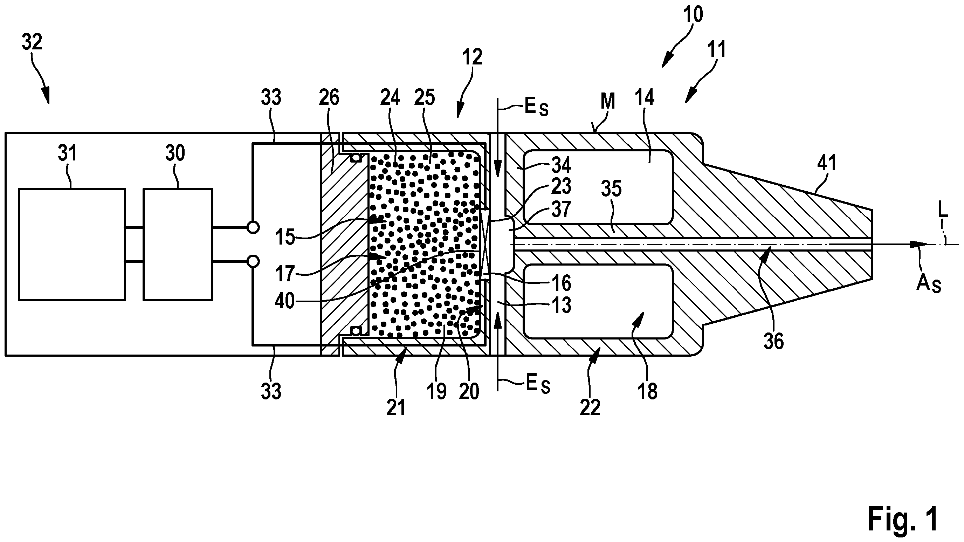

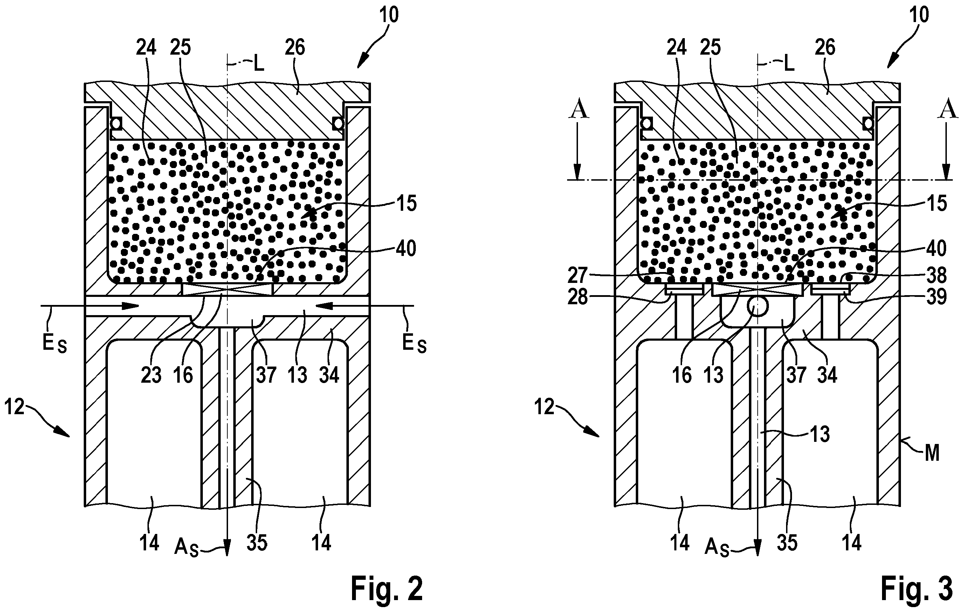

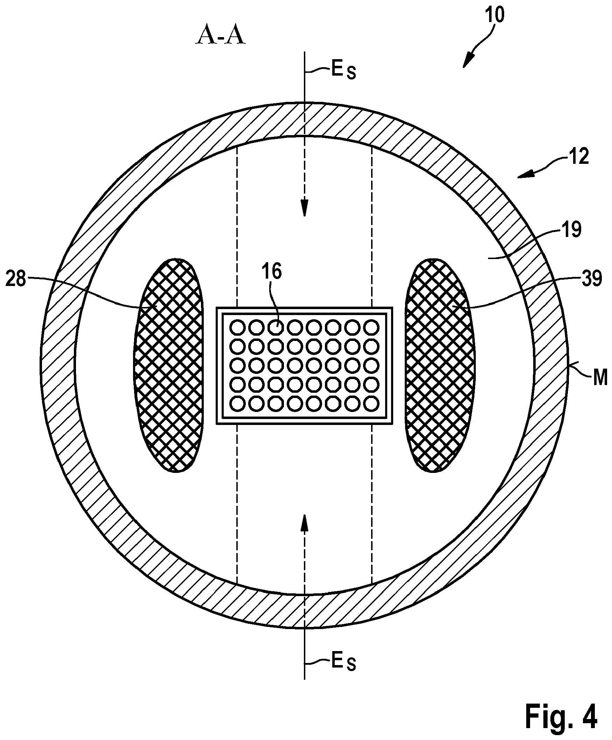

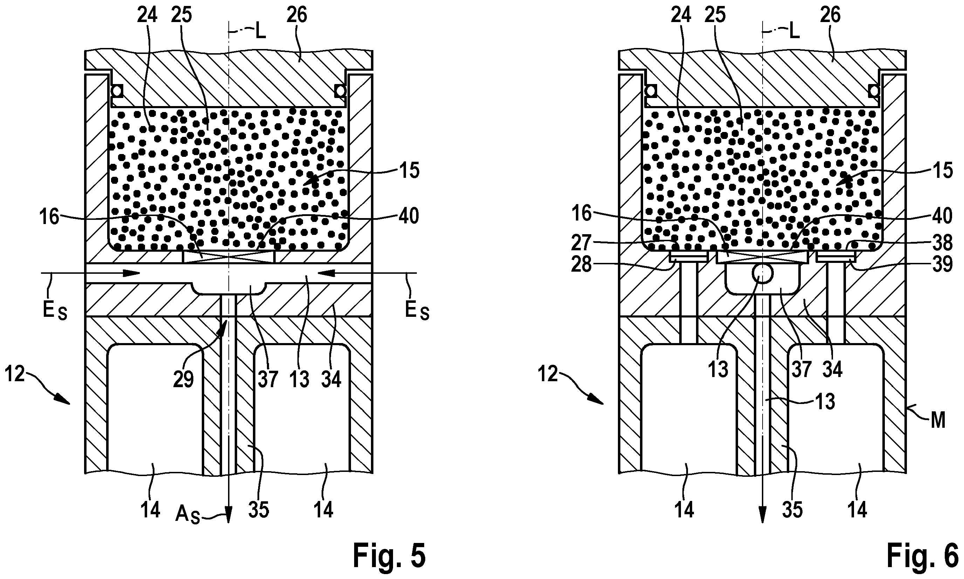

Vorzugsweise ist die Verdampfereinheit 17 Bestandteil eines Mehrwegartikels des Inhalators 11, während der Vorratstank 14 Bestandteil eines Einmalartikels des Inhalators 11 ist. Die Verdampferkartusche 10 ist bevorzugt als eigenständiges Modul und Einwegartikel ausgebildet. Die Verdampferkartusche 10 kann aber auch ganz oder teilweise zusammen mit anderen Komponenten ein Modul bilden. Bevorzugt ist die Verdampferkartusche 10 zum mechanischen und elektrischen Verbinden mit einem mindestens eine elektronische Steuereinheit 30 und eine Energiequelle 31 umfassenden Kartuschenträger 32 zur Bildung eines Inhalators 11 ausgebildet und eingerichtet, wobei die Verdampfereinheit 17 elektrische Kontakte 33 zur elektrischen Kontaktierung mit der Energiequelle 31 umfasst. Der Inhalator 14 kann z.B. durch eine inhalierende Person aktiviert werden, beispielsweise als E-Zigarette, oder z.B. durch eine Pumpe aktiviert werden, z.B. als medizinisches Instrument für den Fall, das die Person selbst nicht mehr oder nicht ausreichend saugen kann. In der Ausführungsform gemäß der Figuren 1 bis 4 weist der Kartuschenträger 32 als Mehrwegartikel nur die Steuereinheit 30 und die Energiequelle 31 auf. Die Verdampfereinheit 17 und der Vorratstank 14 sind Bestandteil der Verdampferkartusche 10 als Einmal- oder Wegwerfartikel. In den Figuren 5 und 6 besteht der Einmalartikel ausschließlich aus dem Vorratstank 14. Durch Zusammenfügen, Aufstecken oder dergleichen des Vorratstanks 14 als Einmalartikel mit der bzw. an die Verdampfereinheit 17, die Bestandteil des Kartuschenträgers 32 als Mehrwegartikel ist, bildet sich die Verdampfereinheit 17.

Wie bereits erwähnt, umfasst die Verdampferkartusche 10 einen durchgängigen Strömungskanal 13 mit mindestens einer Eintrittsseite Es und mindestens einer Austrittsseite As, wobei der Begriff durchgängig beschreibt, dass der Strömungskanal 13 an der oder jeder Eintrittsseite Eₛ und an der oder jeder Austrittsseite Aₛ luftdurchlässig ist. In den Figuren sind vorzugsweise zwei Eintrittsseiten Es in den Strömungskanal 13 ausgebildet. Optional können aber auch nur eine Eintrittsseite Es oder mehr als zwei Eintrittsseiten Es vorgesehen und ausgebildet sein. Der Strömungskanal 13 bzw. der Strömungskanalabschnitt, der in der Zwischenwand 20 ausgebildet ist und radial gerichtet ist, also quer zur Längsachse L des Gehäusekörpers 12 verläuft, kann sich z.B. in der Mantelfläche M des Gehäusekörpers 12 parallel zur Längsachse L fortsetzen, vorzugsweise bis zum Austrittsseite As auf der dem Deckelelement 26 gegenüberliegenden Stirnseite. Bevorzugt weist die Zwischenwand 20 ausgehend von einem den Hohlraum 18 teilenden, tellerförmigen Abschnitt 34 mit einem quer zur Längsachse L des Hohlraums 18 bzw. des Gehäusekörpers 12 verlaufenden Abschnitt des Strömungskanals 13 einen durch den Vorratstank 14 verlaufenden kanalartigen Abschnitt 35 mit einem vorzugsweise parallel zur Längsachse L des Hohlraums 18 bzw. des Gehäusekörpers 12 verlaufenden Abschnitt des Strömungskanals 13 zur Bildung eines Schlots 36 auf, wobei die Abschnitte des Strömungskanals 13 miteinander verbunden sind und in ihrer Schnittstelle eine Dampfkammer 37 bilden.

Vorzugsweise verläuft der parallel zur Längsachse L des Hohlraums 18 bzw. des Gehäusekörpers 12 verlaufende kanalartige Abschnitt 35 der Zwischenwand 20 zentral durch den Vorratstank 14, wobei zu beiden Seiten des kanalartigen Abschnitts 35 der Zwischenwand 20 im tellerförmigen Abschnitt 34 derselben jeweils eine Zugangsöffnung 27, 38 zur Herstellung einer Fluidverbindung vom Vorratstank 14 zum Aufnahmeraum 19 ausgebildet ist, die mit einer flüssigkeitspermeablen Gitterstruktur 28, 39 abgedeckt ist. Der Strömungskanal 13 kann sich aber auch vom Deckelelement 26 bis zu einem Mundstück 41 erstrecken.

Die Körner 24 liegen als lose Schüttung innerhalb des Aufnahmeraums 19. So können sich die Körner 24 selbst im Betriebszustand der Verdampferkartusche 10 noch relativ zueinander bewegen und so variable Mikrokanäle 25 bilden. Die nebeneinander und übereinander liegenden Körner 24 stützen sich gegeneinander ab. Dabei können die Körner 24 rein mechanisch aneinanderstoßen. Die Körner 24 können aber auch untereinander mechanisch miteinander verzahnt sein. Die Körner 24 des Dochtorgans 15 können hinsichtlich ihrer Materialauswahl und/oder ihrer Größe gleich und/oder ungleich ausgebildet sein. Alle Körner 24 können die gleiche Größe aufweisen, also in einem Größenbereich liegen. Die Körner 24 können jedoch unterschiedliche Größen aufweisen, also in unterschiedlichen Größenbereichen liegen. Bevorzugt beträgt die Korngröße zwischen 0,1µm und 2mm und besonders bevorzugt zwischen 3µm und 300µm. Rein beispielhaft können alle Körner 24 in einem Größenbereich zwischen 50µm und 100µm (entspricht einem Größenbereich) liegen. Die Körner 24 des Dochtorgans 15 können aber auch lokal unterschiedliche Korngrößen aufweisen. Durch die Wahl der Korngrößen und der jeweiligen Verteilung z.B. in Schichten mit Körnern 24 unterschiedlicher Größenbereiche lässt sich u.a. der Strömungswiderstand des Dochtorgans 15 individuell, letztlich sogar auch erst bei der Schüttung, einstellen. Der minimale Korndurchmesser der Körner 24 im feinporigen Bereich sollte dabei vorzugsweise größer sein, als die Poren im nächst gröberen Bereich, um den Porengradient stabil zu halten. Durch die Auswahl der eingesetzten Korngrößen in einem Dochtorgan 15 lässt sich ein individueller Porengradient für das Dochtorgan 15 einstellen. Die maximale Korngröße liegt in Abhängigkeit der Fließeigenschaft der jeweils zu fördernden Flüssigkeit jeweils außerhalb einer die kapillare Förderung ausschließenden Größe. Anders ausgedrückt dürfen die Körner 24 nur so groß sein, dass sie als Dochtorgan 15 noch eine kapillare Wirkung erzeugen. In gleichem Maße darf der Korndurchmesser nicht kleiner als der Durchmesser der Kapillaren bzw. Poren des Heizorgans 16 ausgeprägt sein, um ein Verstopfen des Heizorgans 16 bzw. ein Austreten der Körner 24 aus der Heizstruktur zu vermeiden.

Alle Körner 24 können aus demselben Material bestehen. Die Körner 24 können jedoch auch aus wenigstens zwei unterschiedlichen Materialien bestehen. Vorzugsweise bestehen die Körner 24 aus Sand (Quarz) und/oder Graphit. Als Materialien kommen aber auch diverse andere Materialien oder Materialmischungen in Frage. Bevorzugte Materialien für die Körner 24 sind z.B. PEEK-Granulat (Polyetheretherketon-Granulat), PEK-Granulat (Polyetherketon-Granulat), PA-Pulver, VM17-Granulat, Glas, Steatit, Siliziumdioxid, Lignin, Aerogel, Viton, Silikon, Asche, Charcoal, Betonit, Zeolith, Diatomit, Magnesiumsilikat, Korund, Kieselgur, gemahlener Porphyr sowie Mischungen daraus. Besonders bevorzugt bestehen die Körner 24 eines Dochtorgans 15 lokal aus unterschiedlichen Materialien. Als lokale Anordnung ist z.B. ein schichtweiser Aufbau von Körnern 24 aus jeweils gleichem Material zu verstehen.

Durch die Auswahl der Materialien der Körner 24 eines Dochtorgans 15 lassen sich verschiedene Eigenschaften des Dochtorgans 15 einstellen. Beispielsweise können Körner 24 mit unterschiedlichen Wärmeleitfähigkeiten eingesetzt werden. Die unterschiedliche Materialauswahl der Körner 24 führt auch dazu, dass die Körner 24 z.B. kompressibel ausgebildet sein können. In Abhängigkeit der Größe des Anpressdruckes, mit dem die Körner 24 z.B. in dem Aufnahmeraum 19 gehalten werden, kann durch elastische Verformung aktiv Einfluss auf die Größe der Poren einzelner Körner 24 oder benachbarter Körner 24 genommen werden. Der Verdampferkartusche 10 kann optional ein Stellorgan zugeordnet sein, mittels dem im Betriebszustand der Verdampferkartusche 10 der Anpressdruck auf das Dochtorgan 15 einstellbar ist. Das Stellorgan kann z.B. ein Hebelelement, ein Drehelement oder jedes andere Pressmittel sein.

Es lassen sich auch mehrschichtige Dochtorgane 15 bilden. In einer Ausführungsform kann eine erste Schicht mit Körnern 24 einer ersten Kornart gebildet sein. Eine zweite Schicht ist mit Körnern 24 einer zweiten Kornart gebildet. Eine dritte Schicht ist wieder mit der ersten Kornart gebildet. Die Körner 24 der zweiten Kornart in der mittleren Schicht weisen eine spezifische Eigenschaft auf, die z.B. mittels eines Mikrocontrollers der Steuereinheit 30 detektierbar ist. Während des Betriebs der Verdampferkartusche 10 führt z.B. eine Änderung der Benetzung der Körner 24 in der zweiten Schicht zu einer detektierbaren Änderung der spezifischen Eigenschaft der zweiten Kornart. Über den Mikrocontroller, der z.B. ein Sensor sein kann, wird diese Änderung detektiert. Mittels der Steuereinheit 30 kann dann in den Verdampfungsprozess regelnd eingegriffen werden, um z.B. einen so genannten Dry-Puff des Heizorganes 16 zu verhindern.

Die Körner 24 des Dochtorgans 15 können gleiche oder unterschiedliche geometrische Formen aufweisen. Die Körner 24 können beispielsweise nadelförmig, kugelförmig, in der Form eines Reiskorns oder auch dreieckig sein. Die Körner 24 können abgerundete Kanten aufweisen oder scharfkantig ausgebildet sein. Unter dem Begriff "Körner" werden ausdrücklich keine faserigen Elemente verstanden, also keine dünnen, feinen, fadenförmigen Gebilde. In Abhängigkeit der jeweiligen Form der Körner 24 und deren Korngröße können z.B. longitudinale und/ oder sphärische Poren gebildet sein. Die Poren können auch unregelmäßig ausgebildet sein. Die Körner 24 können auch mindestens teilweise magnetisch sein. Dadurch lassen sich die Körner 24 z.B. während des Befüllens/Schüttens in den Aufnahmeraum 19 durch Anlegen eines externen Magnetfeldes in gewünschte Ausrichtungen ausrichten. Mit der Möglichkeit der Ausrichtung der Körner 24, z.B. können nadelförmige Körner 24 senkrecht zum Strömungskanal 13 ausgerichtet werden, lassen sich die Eigenschaften des Dochtorgans 15 individuell bestimmen, um das Dochtorgan 15 z.B. als Rückschlagventil oder als Steuerventil einsetzen zu können.

Das Dochtorgan 15, das nahezu spalt- und hohlraumfrei an dem Heizorgan 16 anliegt, weist Mikrokanäle 23 auf. Das Heizorgan 16 ist flüssigkeits- und gas- bzw. dampfpermeabel ausgebildet. Mit seiner Austrittsseite liegt das Dochtorgan 15 an der Eintrittsseite des Heizorganes 16 an und bildet eine Kontaktfläche 40. Das Heizorgan 16 selbst weist vorzugsweise lineare und/oder nicht lineare Durchgänge auf, die in den Strömungskanal 13 münden. Das Heizorgan 16 kann eine ebene oder gekrümmte oder anderweitig geformte Ausbildung aufweisen. Besonders bevorzugt ist das Heizorgan 16 ein im Wesentlichen aus Silizium bestehendes oder Silizium oder p- oder n-dotiertes Silizium aufweisendes MEMS-Bauteil (Micro-Electro-Mechanical-System), das ausgehend von einer dem Dochtorgan 15 zugewandten Oberseite bis zu einer dem Strömungskanal 13 zugewandten Unterseite flüssigkeits- und gas- bzw. dampfpermeable Durchgänge aufweist. Die minimale Korngröße der Körner 24 des Dochtorgans 15 ist mindestens im Kontaktbereich 40 zum Heizorgan 16 größer ist als der mittlere Durchmesser der Durchgänge des Heizorganes 16.

Das Funktionsprinzip des erfindungsgemäßen Inhalators 11, der eine Verdampferkartusche 10 gemäß der Erfindung umfasst, wird beispielhaft anhand einer E-Zigarette als Inhalator 11 insbesondere mit Bezug auf die Figur 1 beschrieben. Eine konsumierende Person saugt z.B. an einem Mundstück 41 des Inhalators 11, der aus dem Kartuschenträger 32 und der Verdampferkartusche 10 gebildet ist, wobei sich in dem Vorratstank 14 der Verdampferkartusche 10 eine Flüssigkeit befindet, die beispielsweise Glycerin, Propylenglycol und ggf. weitere Wirkstoffe und/oder Geschmacksstoffe enthält. Durch das Saugen wird in dem Strömungskanal 13 ein Unterdruck erzeugt, der seinerseits z.B. über einen nicht dargestellten Sensor die Steuereinheit 30 aktiviert. Die Steuereinheit 30 steuert das Heizorgan 16, das von der Energiequelle 31 mit Energie versorgt wird. Flüssigkeit aus dem Vorratstank 14 wird mittels des Dochtorgans 15 mindestens initial kapillar durch die Mikrokanäle 23 aus dem Vorratstank 14 in Richtung des Heizorganes 16 transportiert. Am bzw. im erwärmten Heizorgan 16 wird die Flüssigkeit zu Gas bzw. Dampf umgewandelt, wobei das Heizorgan 16 die Flüssigkeit bzw. den daraus gebildeten Dampf und/oder Aerosole aufgrund der flüssigkeits- und gas- bzw. dampfpermeablen Struktur in Richtung des Strömungskanals 13 transportiert und an diesen abgibt. Das Gas bzw. der Dampf und/oder Aerosole bilden sich bei der Mischung des Luftstroms im Strömungskanal 13 und werden von der konsumierenden Person angesaugt und inhaliert.

## Patentansprüche

1. Verdampferkartusche (10) als Bestandteil eines Inhalators (11), umfassend einen Gehäusekörper (12) mit einem Strömungskanal (13), einen Vorratstank (14) zum Bevorraten von Flüssigkeit sowie eine ein Dochtorgan (15) und ein Heizorgan (16) umfassende Verdampfereinheit (17), die flüssigkeitspermeabel ausgebildet ist, derart, dass Flüssigkeit mindestens initial kapillar aus dem Vorratstank (14) durch die Verdampfereinheit (17) in Richtung des Strömungskanals (13) förderbar ist, wobei der Gehäusekörper (12) mit einem Hohlraum (18) ausgebildet ist, wobei der Hohlraum (18) des Gehäusekörpers (12) zur Bildung des Vorratstanks (14) einerseits und zur Bildung eines Aufnahmeraums (19) für das Dochtorgan (15) andererseits durch eine den Strömungskanal (13) mindestens abschnittsweise aufweisende Zwischenwand (20) in zwei Kammern (21, 22) aufgeteilt ist, wobei in der Zwischenwand (20) mindestens eine Zugangsöffnung (23) vom Aufnahmeraum (19) für das Dochtorgan (15) zum Strömungskanal (13) und mindestens eine Zugangsöffnung (27) zur Herstellung einer Flüssigkeitsverbindung vom Vorratstank (14) zum Aufnahmeraum (19) ausgebildet sind, **dadurch gekennzeichnet, dass** die Zugangsöffnung (23) vollständig durch das Heizorgan (16) abgedeckt ist, und in dem Aufnahmeraum (19) eine Vielzahl granulatartiger, lose nebeneinanderliegender Körner (24) zur Bildung des am Heizorgan (16) anliegenden Dochtorgans (15) angeordnet sind, die den Aufnahmeraum (19) vorzugsweise ausfüllen und aufgrund ihrer losen Schüttung und/oder Ausbildung Mikrokanäle (25) bilden und durch ein Deckelelement (26) im Aufnahmeraum (19) gehalten werden, wobei die Zugangsöffnung (27) mit einer flüssigkeitspermeablen Gitterstruktur (28) abgedeckt ist.

2. Verdampferkartusche (10) nach Anspruch 1, **dadurch gekennzeichnet, dass** der Gehäusekörper (12) mit einem durchgängigen Strömungskanal (13) ausgebildet ist, der mindestens eine Eintrittsseite E_{S} und mindestens eine Austrittsseite A_{S} aufweist, wobei der Gehäusekörper (12) rohrförmig mit einem sich längsaxial erstreckenden Hohlraum (18) ausgebildet ist und durch eine den durchgängigen Strömungskanal (13) mindestens abschnittsweise aufweisende Zwischenwand (20) in zwei längsaxial hintereinander angeordnete Kammern (21, 22) aufgeteilt ist.

3. Verdampferkartusche (10) nach Anspruch 1 oder 2, **dadurch gekennzeichnet, dass** die Verdampfereinheit (17) Bestandteil eines Mehrwegartikels des Inhalators (11) ist, während der Vorratstank (14) Bestanteil eines Einmalartikels des Inhalators (11) ist.

4. Verdampferkartusche (10) nach einem oder mehreren der Ansprüche 1 bis 3, **dadurch gekennzeichnet, dass** die Zwischenwand (20) ausgehend von einem den Hohlraum (18) teilenden, tellerartigen Abschnitt (34) mit einem vorzugsweise quer zur Längsachse des Hohlraums (18) verlaufenden Abschnitt des Strömungskanals (13) einen durch den Vorratstank (14) verlaufenden kanalartigen Abschnitt (35) mit einem vorzugsweise parallel zur Längsachse des Hohlraums (18) verlaufenden Abschnitt des Strömungskanals (13) zur Bildung eines Schlots (36) aufweist, wobei die beiden Abschnitte des Strömungskanals (13) miteinander verbunden sind und in ihrer Schnittstelle eine Dampfkammer (37) bilden.

5. Verdampferkartusche (10) nach Anspruch 4, **dadurch gekennzeichnet, dass** der vorzugsweise parallel zur Längsachse des Hohlraums (18) verlaufende kanalartige Abschnitt (35) der Zwischenwand (20) zentral durch den Vorratstank (14) verläuft, wobei zu beiden Seiten des kanalartigen Abschnitts (35) der Zwischenwand (20) im tellerartigen Abschnitt (34) derselben jeweils eine Zugangsöffnung (27, 38) zur Herstellung einer Fluidverbindung vom Vorratstank (14) zum Aufnahmeraum (19) ausgebildet ist, die mit einer flüssigkeitspermeablen Gitterstruktur (28, 39) abgedeckt ist.

6. Verdampferkartusche (10) nach einem oder mehreren der Ansprüche 1 bis 5, **dadurch gekennzeichnet, dass** das Heizorgan (16) ein im Wesentlichen aus Silizium bestehendes oder Silizium oder p- oder n-dotiertes Silizium aufweisendes MEMS-Bauteil (Micro-Electro-Mechanical-System) ist, das ausgehend von einer dem Dochtorgan (15) zugewandten Oberseite bis zu einer dem Strömungskanal (13) zugewandten Unterseite flüssigkeits- und gas- bzw. dampfpermeable Durchgänge aufweist.

7. Verdampferkartusche (10) nach einem oder mehreren der Ansprüche 1 bis 6, **dadurch gekennzeichnet, dass** sie zum mechanischen und elektrischen Verbinden mit einem mindestens eine elektronische Steuereinheit (30) und eine Energiequelle (31) umfassenden Kartuschenträger (32) zur Bildung eines Inhalators (11) ausgebildet und eingerichtet ist, wobei die Verdampfereinheit (17) elektrische Kontakte (33) zur elektrischen Kontaktierung mit der Energiequelle (31) umfasst.

8. Inhalator (11), ausgebildet und eingerichtet zum Inhalieren von mit Wirkstoffen angereichertem Dampf, umfassend einen mindestens eine elektronische Steuereinheit (30) und eine Energiequelle (31) umfassenden Kartuschenträger (32) sowie eine Verdampferkartusche (10), **dadurch gekennzeichnet, dass** die Verdampferkartusche (10) nach einem oder mehreren der Ansprüche 1 bis 7 ausgebildet und eingerichtet ist.

## Claims

1. Vaporizer cartridge (10) as a component of an inhaler (11), comprising a housing body (12) with a flow channel (13), a storage tank (14) for storing liquid as well as a vaporizer unit (17) comprising a wick member (15) and a heating member (16), which vaporizer unit (17) is formed to be liquid-permeable in such a manner that liquid can be conveyed at least initially in a capillary manner from the storage tank (14) through the vaporizer unit (17) in the direction of the flow channel (13), wherein the housing body (12) is formed with a cavity (18), wherein the cavity (18) of the housing body (12) for the formation of the storage tank (14) on the one hand and for the formation of a receiving chamber (19) for the wick member (15) on the other hand is divided by an intermediate wall (20) having the flow channel (13) at least in sections into two chambers (21, 22), wherein at least one access opening (23) from the receiving chamber (19) for the wick member (15) to the flow channel (13) and at least one access opening (27) for the establishment of a liquid connection from the storage tank (14) to the receiving chamber (19) is formed in the intermediate wall (20), **characterized in that** the access opening (23) is entirely covered by the heating member (16), and a plurality of granular grains (24) lying loosely next to each other for the formation of the wick member (15) bearing against the heating member (16) are arranged in the receiving chamber (19), which plurality of granular grains (24) preferably fill out the receiving chamber (19) and as a result of their loose fill and/or formation form microchannels (25) and are retained in the receiving chamber (19) by a cover element (26), wherein the access opening (27) is covered with a liquid-permeable grid structure (28).

2. Vaporizer cartridge (10) according to Claim 1, **characterised in that** the housing body (12) is formed with a continuous flow channel (13), which has at least one entry side E_{S} and at least one exit side A_{S}, wherein the housing body (12) is formed to be tubular with a cavity (18) extending in a longitudinally axial manner and is divided by an intermediate wall (20) having the continuous flow channel (13) at least in sections (20) into two chambers (21, 22) arranged consecutively in a longitudinally axial manner.

3. Vaporizer cartridge (10) according to Claim 1 or 2, **characterised in that** the vaporizer unit (17) is a component of a multi-use article of the inhaler (11), while the storage tank (14) is a component of a single-use article of the inhaler (11).

4. Vaporizer cartridge (10) according to one or more of Claims 1 to 3, **characterised in that** the intermediate wall (20) has, proceeding from a plate-like section (34) which divides the cavity (18), with a section of the flow channel (13) running preferably transverse to the longitudinal axis of the cavity (18), a channel-like section (35) running through the storage tank (14) with a section of the flow channel (13) running preferably parallel to the longitudinal axis of the cavity (18) for the formation of a vent (36), wherein the two portions of the flow channel (13) are connected to one another and form a vapour chamber (37) at their interface.

5. Vaporizer cartridge (10) according to Claim 4, **characterised in that** the channel-like section (35) of the intermediate wall (20) running preferably parallel to the longitudinal axis of the cavity (18) runs centrally through the storage tank (14), wherein in each case an access opening (27, 38) for establishing a fluid connection from the storage tank (14) to the receiving chamber (19) is formed on both sides of the channel-like section (35) of the intermediate wall (20) in the plate-like section (34) thereof, which access opening (27, 38) is covered with a liquid-permeable grid structure (28, 39).

6. Vaporizer cartridge (10) according to one or more of Claims 1 to 5, **characterised in that** the heating member (16) is a MEMS component (Micro-Electro-Mechanical-System) which is composed substantially of silicon or has silicon or p- or n-doped silicon and, proceeding from an upper side facing the wick member (15) down to a lower side facing the flow channel (13), has liquid- and gas- or vapour-permeable passages.

7. Vaporizer cartridge (10) according to one or more of Claims 1 to 6, **characterised in that** it is configured and adapted for mechanical and electrical connection to a cartridge carrier (32), for the formation of an inhaler (11), at least comprising an electronic control unit (30) and an energy source (31), wherein the vaporizer unit (17) comprises electrical contacts (33) for electrical contact with the energy source (31).

8. Inhaler (11), configured and adapted for the inhalation of vapour enriched with active ingredients, comprising a cartridge carrier (32) at least comprising an electronic control unit (30) and an energy source (31) as well as a vaporizer cartridge (10), **characterised in that** the vaporizer cartridge (10) is configured and adapted according to one or more of Claims 1 to 7.

## Revendications

1. Cartouche de vaporisation (10) faisant partie d'un inhalateur (11), comprenant un corps de boîtier (12) avec un canal d'écoulement (13), un réservoir (14) pour stocker du liquide ainsi qu'une unité de vaporisation (17) comportant un organe de mèche (15) et un organe chauffant (16) et étant agencée perméable au liquide de façon que du liquide puisse être transporté, au moins initialement de façon capillaire, du réservoir (14) par l'unité de vaporisation (17) en direction du canal d'écoulement (13), le corps de boîtier (12) étant formé avec un espace creux (18), l'espace creux (18) du corps de boîtier (12) étant divisé en deux chambres (21, 22) pour former le réservoir (14), d'une part, et pour former un espace de réception (19) pour l'organe de mèche (15), d'autre part, par une paroi intermédiaire (20) comprenant le canal d'écoulement (13) au moins par sections, au moins une ouverture d'accès (23) de l'espace de réception (19) pour l'organe de mèche (15) vers le canal d'écoulement (13) et au moins une ouverture d'accès (27) pour établir une liaison pour liquide du réservoir (14) à l'espace de réception (19) étant formée dans la paroi intermédiaire (20),
**caractérisée en ce que** l'ouverture d'accès (23) est entièrement recouverte par l'organe chauffant (16) et **en ce qu'**une pluralité de graines (24) en forme de granulat disposées librement les unes à côté des autres, sont disposées dans l'espace de réception (19) pour former l'organe de mèche (15) en contact avec l'organe chauffant (16) et qui, de préférence, remplissent l'espace de réception (19) et qui, de par leur disposition libre et/ou leur configuration, forment des micro-canaux (25) et sont maintenues dans l'espace de réception (19) par un élément de couvercle (26), l'ouverture d'accès (27) étant recouverte d'une structure de grille (28) perméable aux liquides

2. Cartouche de vaporisation (10) selon la revendication 1, **caractérisée en ce que** le corps de boîtier (12) est formé avec un canal d'écoulement (13) de passage qui comprend au moins un côté d'admission Es et au moins un côté de sortie As, le corps de boîtier (12) ayant une forme tubulaire avec un espace creux (18) s'étendant dans la direction de l'axe longitudinal, et étant divisé en deux chambres (21, 22), disposées l'une après l'autre en direction de l'axe longitudinal, par une paroi intermédiaire (20) comprenant, au moins par section, un canal d'écoulement (13) de passage.

3. Cartouche de vaporisation (10) selon la revendication 1 ou 2, **caractérisée en ce que** l'unité de vaporisation (17) est une partie intégrante d'un article à usage multiple de l'inhalateur (11) alors que le réservoir (14) est une partie intégrante d'un article à usage unique de l'inhalateur (11).

4. Cartouche de vaporisation (10) selon une ou plusieurs des revendications 1 à 3, **caractérisée en ce que** la paroi intermédiaire (20) comprend, à partir d'une section (34) en forme d'assiette divisant l'espace creux (18), ensemble avec une section du canal d'écoulement (13) s'étendant de préférence transversalement à l'axe longitudinal de l'espace creux (18), une section (35) en forme de canal passant par le réservoir (14) avec une section du canal d'écoulement (13) s'étendant de préférence parallèlement à l'axe longitudinal de l'espace creux (13) pour la formation d'une cheminée (36), les deux sections du canal d'écoulement (13) étant reliées l'une à l'autre et formant, à leur jonction, une chambre de vapeur (37).

5. Cartouche de vaporisation (10) selon la revendication 4, **caractérisée en ce que** la section (35) de la paroi intermédiaire (20), en forme de canal passant de préférence parallèlement à l'axe longitudinal de l'espace creux (18), passe de manière centrale par le réservoir (14), des deux côtés de la section (35) en forme de canal de la paroi intermédiaire (20), dans la section (34) en forme d'assiette de celle-ci, une ouverture d'admission (27, 38) respective étant formée pour établir une liaison fluidique du réservoir (14) à l'espace de réception (19), ouverture qui est recouverte d'une structure de grille (28, 39) perméable aux liquides.

6. Cartouche de vaporisation (10) selon une ou plusieurs des revendications 1 à 5, **caractérisée en ce que** l'organe chauffant (16) est un composant MEMS (Micro-Electro-Mechanical-System) constitué essentiellement de silicium ou comprenant du silicium ou du silicium à dopage p ou à dopage n, qui comprend des passages perméables aux liquides, aux gaz et à la vapeur, allant d'un côté supérieur orienté vers l'organe de mèche (15) jusqu'à un côté inférieur orienté vers le canal d'écoulement (13).

7. Cartouche de vaporisation (10) selon une ou plusieurs des revendications 1 à 6, **caractérisée en ce qu'**elle est formée et agencée, en vu de former un inhalateur (11), pour être reliée mécaniquement et électriquement à un porte-cartouche (32) comprenant au moins une unité de commande électronique (30) et une source d'énergie (31), l'unité d'évaporation (17) comprenant des contacts électriques (33) pour établir un contact électrique avec la source d'énergie (31).

8. Inhalateur (11) configuré et agencé pour l'inhalation d'une vapeur enrichie de substances actives, comprenant un porte-cartouche (32) comprenant au moins une unité de commande électronique (30) et une source d'énergie (31) ainsi qu'une cartouche de vaporisation (10), **caractérisé en ce que** la cartouche de vaporisation (10) est configurée et agencée selon une ou plusieurs des revendications 1 à 7.
